# EUROPEAN PATENT APPLICATION

(11) **EP 4 733 392 A1**
(43) Date of publication of application: **29.04.2026**
(21) Application number: 23942481.5
(22) Date of filing: 24.11.2023
(51) Int. Cl.: C12N 5/0775, C12N 5/077

(54) **MEDIUM FOR CULTURING STEM CELLS, CELL-PRODUCED PROTEIN COMPOSITION, AND METHOD FOR INDUCING DIFFERENTIATION INTO OSTEOBLAST PRECURSOR CELLS OR OSTEOBLASTS**

(30) Priority: 20.06.2023 JP 2023101035
(71) Applicant: Biofuture Technologies Ltd., Tokyo 105-0014 (JP); Maruta, Koichiro, Fukuoka City, Fukuoka 810-0041 (JP)
(72) Inventor: ISHIZUKA, Yasuyuki, Tokyo 105-0014 (JP); MARUTA, Koichiro, Fukuoka City, Fukuoka 810-0041 (JP)
(74) Representative: Murgitroyd & Company
(86) International application number: PCT/JP2023/042255
(87) International publication number: WO 2024/262053

(57) **Abstract**

Mesenchymal stem cells and osteoblast progenitor cells are induced to differentiate into osteoblasts. Provided is a culture medium for stem cell culture containing 60 vol% to 90 vol% of a basal culture medium and 10 vol% to 40 vol% of physiological saline, and further having, added thereto, 1 ng/mL to 100 ng/mL of EGF, 0.2 ng/mL to 20.0 ng/mL of FGF-2, 0.2 ng/mL to 20.0 ng/mL of PDGF, and 0.5 mM to 8.0 mM of magnesium ascorbyl phosphate.

## Description

### TECHNICAL FIELD

The present disclosure relates to a culture medium for stem cell culture, a cell producing proteins composition that is obtained using this culture medium for stem cell culture, and a method of inducing differentiation into osteoblasts and a method of inducing differentiation into osteoblast progenitor cells that use this cell producing proteins composition, and, in particular, provides a method of inducing differentiation of mesenchymal stem cells or osteoblast progenitor cells into osteoblasts through a cell producing proteins composition that has been obtained using a culture medium for stem cell culture.

### BACKGROUND

Bone metabolism is maintained through a balance between osteoblasts that make bone and osteoclasts that absorb bone. Osteoblasts are derived from mesenchymal stem cells, and osteoclasts are thought to be derived from modeling monocytes/macrophages.

A known conventional method of causing differentiation of mesenchymal stem cells into osteoblasts involves culturing mesenchymal stem cells (MSCs) in a dish or plate containing a culture medium for cell proliferation, switching the culture medium to an osteoblast differentiation-inducing culture medium at a point at which a state of 80% confluence or higher is reached, and then culturing for 2 weeks. Moreover, a method in which calcification arising through induction of differentiation of mesenchymal stem cells into osteoblasts is evaluated by staining calcium with a pigment such as Alizarin Red is known as an index for evaluating induction of differentiation into osteoblasts (for example, Patent Literature (PTL) 1).

### CITATION LIST

### Patent Literature

PTL 1: JP 2005-124460 A

### SUMMARY

### (Technical Problem)

However, with a method such as described above, calcification occurs as a result of mesenchymal stem cells being induced to differentiate into osteoblasts, which makes it difficult to detach cells from a dish or plate, and thus it has not been possible to perform reseeding, administration, transplantation, or the like of differentiated osteoblasts at a target location.

On the other hand, the formation of nodules (calcified spheres) indicating the start of calcification has been used to judge an osteoblast progenitor cell stage, which is a differentiated state at a prior stage to osteoblasts, and then collection and reseeding, administration, transplantation, or the like have been performed at this osteoblast progenitor cell stage. However, in this situation, calcification is not observed at a reseeding, administration, or transplantation site, and osteoblast progenitor cells return to an undifferentiated state.

This demonstrates that differentiation of osteoblast progenitor cells into osteoblasts does not proceed when osteoblast progenitor cells are simply reseeded, administered, transplanted, etc. without an osteoblast differentiation-inducing culture medium.

Moreover, although the use of an osteoblast differentiation-inducing culture medium at a reseeding, administration, or transplantation site enables induction of differentiation into osteoblasts to proceed in around 10 days, it is difficult to administer/supplement an osteoblast differentiation-inducing culture medium at an administration/transplantation site for around 10 days, particularly when the administration/transplantation site is in vivo.

Dexamethasone, ascorbic acid, and β-glycerophosphoric acid are thought to be necessary for inducing differentiation of mesenchymal stem cells into osteoblasts, and these components are contained in osteoblast differentiation-inducing culture media.

Moreover, fetal bovine serum (FBS), which has strong action of causing differentiation of mesenchymal stem cells into osteoblasts and does not contain components displaying cell toxicity, is typically used in osteoblast differentiation-inducing culture media. Since FBS is a component derived from a different animal species, it is difficult to use FBS at an administration/transplantation site such as described above due to the risk of antigenicity and zoonotic virus infection.

As a result of further studies conducted by the inventors in light of the circumstances set forth above, the inventors reached a new finding of cell producing proteins (CPPs) containing osteoblast differentiation-inducing components that can be used instead of an osteoblast differentiation-inducing culture medium and that can be produced from mesenchymal stem cells, thus leading to the present disclosure.

The present disclosure was completed in light of the circumstances set forth above and is directed at inducing differentiation of mesenchymal stem cells and osteoblast progenitor cells into osteoblasts. More specifically, the present disclosure is directed at the provision of a method of inducing differentiation into osteoblasts that makes it possible to cause differentiation of mesenchymal stem cells and osteoblast progenitor cells into osteoblasts using components obtained from mesenchymal stem cells, without using an osteoblast differentiation-inducing culture medium.

### (Solution to Problem)

The present disclosure was completed based on the finding described above and is directed at advantageously solving the problems set forth above. A first aspect of the present disclosure is a culture medium for stem cell culture comprising:
60 vol% to 90 vol% of a basal culture medium; and
10 vol% to 40 vol% of physiological saline, and further having, added thereto:
   1 ng/mL to 100 ng/mL of EGF;
   0.2 ng/mL to 20.0 ng/mL of FGF-2;
   0.2 ng/mL to 20.0 ng/mL of PDGF; and
   0.5 mM to 8.0 mM of magnesium ascorbyl phosphate.

Through a culture medium for stem cell culture such as set forth above, it is possible to produce a cell producing proteins (CPPs) composition from mesenchymal stem cells and to induce differentiation of mesenchymal stem cells and osteoblast progenitor cells into osteoblasts through this CPPs composition.

IMDM, DMEM, or α-MEM can be used as the basal culture medium. The aim of adding physiological saline in the composition of the culture medium for stem cell culture is to lower the Ca concentration in the basal culture medium to 10% to 40%. Therefore, dilution with physiological saline is not necessary in a situation in which the Ca concentration in the used basal culture medium is 40 µg/mL to 50 µg/mL.

A second aspect of the present disclosure is a cell producing proteins (CPPs) composition for inducing differentiation of mesenchymal stem cells or osteoblast progenitor cells into osteoblasts, wherein the CPPs composition is obtained by:
culturing mesenchymal stem cells in the culture medium for stem cell culture according to the first aspect for 1 to 10 days;
subsequently causing sedimentation of protein components in an organic solvent; and
separating a sedimented portion and dissolving the sedimented portion in physiological saline or a basal culture medium.

Through a CPPs composition such as set forth above, it is possible to induce differentiation of mesenchymal stem cells and osteoblast progenitor cells into osteoblasts using components obtained from mesenchymal stem cells, without using an osteoblast differentiation-inducing culture medium.

In the second aspect set forth above, the CPPs composition may comprise type I procollagen and hyaluronic acid, and calcium may be bonded to the type I procollagen.

By forming a composite with calcium, type I procollagen can serve as a cell scaffolding and can facilitate the supply of calcium to cells.

A third aspect of the present disclosure is a method of inducing differentiation into osteoblasts comprising:
causing differentiation of mesenchymal stem cells into osteoblast progenitor cells by
   1) culturing the mesenchymal stem cells for 2 to 6 days in an osteoblast differentiation-inducing culture medium with the mesenchymal stem cells in a state adhered to a culture vessel and causing proliferation until confluence or beyond, or
   2) placing the mesenchymal stem cells at rest and performing 2 to 4 days of refrigerated storage with the mesenchymal stem cells in a suspended state in a culture vessel; and
subsequently collecting the osteoblast progenitor cells, seeding the osteoblast progenitor cells in a culture vessel, and culturing the osteoblast progenitor cells in a basal culture medium having the CPPs composition according to the second aspect added thereto to cause differentiation of the osteoblast progenitor cells into osteoblasts.

Through a method of inducing differentiation into osteoblasts such as set forth above, it is possible to induce differentiation of osteoblast progenitor cells into osteoblasts using components obtained from mesenchymal stem cells, without using an osteoblast differentiation-inducing culture medium.

In the aspect set forth above, the mesenchymal stem cells may be derived from bone marrow, adipose tissue, peripheral blood, umbilical cord, umbilical cord blood, or dental pulp.

A fourth aspect of the present disclosure is a method of inducing differentiation into osteoblast progenitor cells comprising causing differentiation of mesenchymal stem cells into osteoblast progenitor cells by:
1) culturing the mesenchymal stem cells for 2 to 6 days in an osteoblast differentiation-inducing culture medium with the mesenchymal stem cells in a state adhered to a culture vessel and causing proliferation until confluence or beyond; or
2) placing the mesenchymal stem cells at rest and performing 2 to 4 days of refrigerated storage with the mesenchymal stem cells in a suspended state in a culture vessel.

Through a method of inducing differentiation into osteoblast progenitor cells such as set forth above, it is possible to prepare osteoblast progenitor cells for reseeding, administration, or transplantation without the occurrence of calcification prior to reseeding, administration, or transplantation.

In the aspect set forth above, the mesenchymal stem cells may be derived from bone marrow, adipose tissue, peripheral blood, umbilical cord, umbilical cord blood, or dental pulp.

A fifth aspect of the present disclosure is a method of inducing differentiation into osteoblasts comprising:
seeding osteoblast progenitor cells that have been obtained by the method of inducing differentiation according to the fourth aspect and simultaneously adding the CPPs composition according to the second aspect to cause differentiation of the osteoblast progenitor cells into osteoblasts; or
seeding osteoblast progenitor cells that have been obtained by the method of inducing differentiation according to the fourth aspect and subsequently adding the CPPs composition according to the second aspect to initiate differentiation of the osteoblast progenitor cells into osteoblasts.

Through a method of inducing differentiation into osteoblasts such as set forth above, it is possible to induce differentiation of osteoblast progenitor cells into osteoblasts at a desired timing at a reseeding, administration, or transplantation site.

### (Advantageous Effect)

According to the present disclosure, it is possible to cause differentiation of mesenchymal stem cells and osteoblast progenitor cells into osteoblasts using components obtained from mesenchymal stem cells, without using an osteoblast differentiation-inducing culture medium.

### BRIEF DESCRIPTION OF THE DRAWINGS

In the accompanying drawings:
FIG. 1 presents (A) a photograph of Alizarin Red staining, (B) measurement results of light absorbance at OD 450 nm, and (C) an enlarged micrograph (×40) of Alizarin Red staining;
FIG. 2 presents results of Example 2;
FIG. 3 presents results of Example 2;
FIG. 4 presents results of Example 2;
FIG. 5 presents results of Example 2;
FIG. 6 presents results of Example 3; and
FIG. 7 presents results of Example 7.

### DETAILED DESCRIPTION

The following provides a detailed description of embodiments of the present disclosure.

### (Culture medium for stem cell culture)

A culture medium for stem cell culture according to a first aspect of the present disclosure contains:
60 vol% to 90 vol% of a basal culture medium; and
10 vol% to 40 vol% of physiological saline, and further has, added thereto:
   1 ng/mL to 100 ng/mL of EGF;
   0.2 ng/mL to 20.0 ng/mL of FGF-2;
   0.2 ng/mL to 20.0 ng/mL of PDGF; and
   0.5 mM to 8.0 mM of magnesium ascorbyl phosphate.

The inventors made a new finding that a cell producing proteins (CPPs) composition described further below is obtained from mesenchymal stem cells that have been cultured using the culture medium for stem cell culture set forth above.

IMDM, DMEM, or α-MEM can be used as the basal culture medium. The aim of adding physiological saline in the composition of the culture medium for stem cell culture is to lower the Ca concentration in the basal culture medium to 10% to 40%. Therefore, dilution with physiological saline is not necessary in a situation in which the Ca concentration in the used basal culture medium is 40 µg/mL to 50 µg/mL.

With regard to additive factors that are added to the culture medium for stem cell culture, EGF is epidermal growth factor, FGF-2 is fibroblast growth factor 2, and PDGF is platelet-derived growth factor. These additive factors are typically used as additive factors in basal culture media with an aim such as causing cell proliferation.

In the culture medium for stem cell culture according to the first aspect of the present disclosure, addition of
1 ng/mL to 10 ng/mL of EGF,
0.5 ng/mL to 5.0 ng/mL of FGF-2, and
0.5 ng/mL to 5.0 ng/mL of PDGF
enables more efficient production of a CPPs composition.

Moreover, in the culture medium for stem cell culture according to the first aspect of the present disclosure, further addition of
0.1 µg/mL to 10.0 µg/mL of transferrin, and/or
0.2 µg/mL to 20.0 µg/mL of insulin, and/or
0.1 ng/mL to 3.0 ng/mL of sodium selenite
is appropriate from a viewpoint of increasing cell proliferation performance.

The specific method by which the culture medium for stem cell culture is produced may, for example, be by
removing 50 mL to 200 mL from 500 mL of IMDM, adding an equal amount of physiological saline to adjust the volume to 500 mL, and adding 5 mL of an antibiotic thereto, and
further adding:
   1 ng/mL to 10 ng/mL of EGF;
   0.5 ng/mL to 5.0 ng/mL of FGF-2;
   0.5 ng/mL to 5.0 ng/mL of PDGF;
   0.1 µg/mL to 10.0 µg/mL of transferrin;
   0.2 µg/mL to 20.0 µg/mL of insulin;
   0.1 ng/mL to 3.0 ng/mL of sodium selenite; and
   0.5 mM to 8.0 mM of magnesium L-ascorbyl phosphate.

### (Cell producing proteins (CPPs) composition)

A CPPs composition according to a second aspect of the present disclosure is a CPPs composition for inducing differentiation of mesenchymal stem cells or osteoblast progenitor cells into osteoblasts and is obtained by:
culturing mesenchymal stem cells in the culture medium for stem cell culture according to the first aspect for 1 to 10 days;
subsequently causing sedimentation of protein components in an organic solvent; and
separating a sedimented portion and dissolving the sedimented portion in physiological saline or a basal culture medium.

Through a CPPs composition such as set forth above, it is possible to induce differentiation of mesenchymal stem cells and osteoblast progenitor cells into osteoblasts. Moreover, through a CPPs composition such as set forth above, it is possible to induce differentiation of mesenchymal stem cells and osteoblast progenitor cells into osteoblasts using components obtained from mesenchymal stem cells and also using components derived from autologous cells, without using an osteoblast differentiation-inducing culture medium.

In the second aspect, the CPPs composition may contain type I procollagen and hyaluronic acid, and calcium may be bonded to the type I procollagen.

For example, the CPPs composition may contain 30 µg/mL to 100 µg/mL of type I procollagen and 49 µg/mL to 140 µg/mL of hyaluronic acid. The bonding ratio of type I procollagen and calcium may be 30% to 45%, for example, with 35% to 42% being appropriate.

In the second aspect, bone marrow-derived mesenchymal stem cells, adipose-derived stem cells (ASCs), peripheral blood-derived mesenchymal stem cells, umbilical cord Wharton's Jelly-derived mesenchymal stem cells, umbilical cord blood mesenchymal stem cells, dental pulp-derived mesenchymal stem cells, or the like can be used as the mesenchymal stem cells. IMDM, DMEM, or α-MEM can be used as the basal culture medium. The culture time is 1 to 10 days, with 3 to 4 days being appropriate.

In the step of "separating a sedimented portion and dissolving the sedimented portion in physiological saline or a basal culture medium" after the step of "causing sedimentation of protein components in an organic solvent" in the second aspect, the protein components that have sedimented in the organic solvent may be suspended in pH 1 hydrochloric acid (for example, 1N HC1), subjected to vortexing (for example, approximately 10 minutes), and then be neutralized (for example, using 1.2N NaOH) so as to also perform virus inactivation treatment. In this situation, the neutralized solution is subjected to centrifugal separation to obtain a supernatant that is then taken to be the cell producing proteins (CPPs) composition.

In the present specification, an "osteoblast progenitor cell" is defined as a "cell where nodules (calcified spheres) indicating the start of calcification have formed (i.e., a partially calcified cell) and that is in a state that is detachable using trypsin-EDTA".

The inventors made a new finding that by culturing mesenchymal stem cells for a short time by step 1) or 2) described below, it is possible to induce differentiation of the mesenchymal stem cells into osteoblast progenitor cells.

### (Method of inducing differentiation of mesenchymal stem cells into osteoblasts)

A method of inducing differentiation into osteoblasts according to a third aspect of the present disclosure includes:
causing differentiation of mesenchymal stem cells into osteoblast progenitor cells by
   1) culturing the mesenchymal stem cells for 2 to 6 days in an osteoblast differentiation-inducing culture medium with the mesenchymal stem cells in a state adhered to a culture vessel and causing proliferation until confluence or beyond, or
   2) placing the mesenchymal stem cells at rest and performing 2 to 4 days of refrigerated storage with the mesenchymal stem cells in a suspended state in a culture vessel; and
subsequently collecting the osteoblast progenitor cells, seeding the osteoblast progenitor cells in a culture vessel, and culturing the osteoblast progenitor cells in a basal culture medium having the CPPs composition according to the second aspect added thereto to cause differentiation of the osteoblast progenitor cells into osteoblasts.

Through a method of inducing differentiation into osteoblasts such as set forth above, it is possible to induce differentiation of osteoblast progenitor cells into osteoblasts using components obtained from mesenchymal stem cells, without using an osteoblast differentiation-inducing culture medium.

In the third aspect, the mesenchymal stem cells may be derived from bone marrow, adipose tissue, peripheral blood, umbilical cord, umbilical cord blood, or dental pulp.

In the third aspect, the concentration of the CPPs composition in the "basal culture medium having the CPPs composition added thereto" is preferably 25 vol% to 75 vol%, and more preferably 50 vol%.

Moreover, "refrigerated storage" means storage at 4°C to 10°C.

Furthermore, "beyond" in "confluence or beyond" means hyperproliferation to a maximum of approximately 200%.

Note that the term "confluence" as used in the present specification means a state in which adherent cells completely cover an adhesion surface of a culture vessel (i.e., a state of 100% confluence).

### (Method of inducing differentiation of mesenchymal stem cells into osteoblast progenitor cells)

A fourth aspect of the present disclosure is a method of inducing differentiation into osteoblast progenitor cells including causing differentiation of mesenchymal stem cells into osteoblast progenitor cells by:
1) culturing the mesenchymal stem cells for 2 to 6 days in an osteoblast differentiation-inducing culture medium with the mesenchymal stem cells in a state adhered to a culture vessel and causing proliferation until confluence or beyond; or
2) placing the mesenchymal stem cells at rest and performing 2 to 4 days of refrigerated storage with the mesenchymal stem cells in a suspended state in a culture vessel.

Through a method of inducing differentiation into osteoblast progenitor cells such as set forth above, it is possible to prepare osteoblast progenitor cells for reseeding, administration, or transplantation without the occurrence of calcification prior to reseeding, administration, or transplantation.

In the fourth aspect, the mesenchymal stem cells may be derived from bone marrow, adipose tissue, peripheral blood, umbilical cord, umbilical cord blood, or dental pulp.

In the fourth aspect, "refrigerated storage" means storage at 4°C to 10°C.

Furthermore, "beyond" in "confluence or beyond" means hyperproliferation to a maximum of approximately 200%.

### (Method of inducing differentiation of osteoblast progenitor cells into osteoblasts)

A fifth aspect of the present disclosure is a method of inducing differentiation into osteoblasts including:
seeding osteoblast progenitor cells that have been obtained by the method of inducing differentiation according to the fourth aspect and simultaneously adding the CPPs composition according to the second aspect to cause differentiation of the osteoblast progenitor cells into osteoblasts; or
seeding osteoblast progenitor cells that have been obtained by the method of inducing differentiation according to the fourth aspect and subsequently adding the CPPs composition according to the second aspect to initiate differentiation of the osteoblast progenitor cells into osteoblasts.

Through a method of inducing differentiation into osteoblasts such as set forth above, it is possible to induce differentiation of osteoblast progenitor cells into osteoblasts at a desired timing at a reseeding, administration, or transplantation site. Particularly in a situation in which the CPPs composition is added after seeding of the osteoblast progenitor cells, the CPPs composition can be used as a booster for initiating differentiation into osteoblasts.

Moreover, in the fifth aspect, the "CPPs composition" is preferably added such as to constitute 25 vol% to 75 vol% of a culture medium that is used to culture the osteoblast progenitor cells, and more preferably constitute 50 vol% of the culture medium.

### EXAMPLES

The following provides a more specific description of the present disclosure through examples. However, the present disclosure is not limited to these examples.

### (Example 1) Production of culture medium for stem cell culture and CPPs composition

A culture medium for stem cell culture that was to be used to produce a CPPs composition was prepared by adding 5 mL of Antibiotic-Antimycotic Mixed Stock Solution (produced by Nacalai Tesque, Inc.; product number: 02892-54) as antibiotics to 500 mL of BSCM-PL2 culture medium for stem cell proliferation (produced by BIOFUTURE TECHNOLOGIES LTD.).

Next, 4 × 10⁵ cells/mL of adipose-derived stem cells (ASCs) (produced by Lonza; product number: PT-2501) categorized as mesenchymal stem cells (MSCs) were cultured to 90% confluence in a T-75 flask (produced by SARSTEDT; product number: 83.3911.002) using BMCM-PL1 culture medium (produced by BIOFUTURE TECHNOLOGIES LTD.) containing 2% of human serum and were detached using trypsin-EDTA solution (produced by Nacalai Tesque, Inc.; product number: 32777-44). Thereafter, 2 × 10⁶ cells/mL were seeded in a HYPERFlask (produced by Corning Incorporated) and were cultured once again to confluence in 560 mL of BMCM-PL1 culture medium (produced by BIOFUTURE TECHNOLOGIES LTD.) containing 1% of human serum.

Thereafter, the culture medium was switched to the BSCM-PL2 culture medium for stem cell proliferation (produced by BIOFUTURE TECHNOLOGIES LTD.). After 5 days of culturing, all of the culture medium was collected, 560 mL of the culture medium for stem cell culture was freshly added, and then a further 5 days of culturing was performed. This total collection and addition of the culture medium was continued until cells detached, and a culture supernatant that was collected in this manner was subjected to centrifugal separation (3,000 rpm, 5 minutes) to remove cell residue. A protein fraction was subsequently separated by organic solvent sedimentation using 80% ethanol or acetone. This protein fraction was dissolved in PBS(-) (produced by Nacalai Tesque, Inc.; product number: 07269-84), was subjected to centrifugal separation (8,000 rpm, 10 minutes) to remove an undissolved fraction, and was then caused to sediment once again using 80% ethanol or acetone.

The sediment was dissolved in 10 mL of 1N hydrochloric acid (produced by Nacalai Tesque, Inc.; product number: 18320-15), was stirred by a vortex at room temperature and 2,500 rpm for 10 minutes, and was neutralized with 1.2N caustic soda (produced by Nacalai Tesque, Inc.; product number: 31511-05) to perform virus inactivation treatment. Thereafter, undissolved matter was removed by centrifugation. Finally, the product was passed through a 0.45 µm filter (produced by Cytiva; product number: 6900-2504) to perform sterilization treatment and obtain a CPPs composition.

### (Comparative Example 1) Production of reference culture supernatant (CM)

Next, a CM that is a typical culture supernatant was produced as follows as a reference for comparison.

4 × 10⁵ cells/mL of ASCs (produced by Lonza; product number: PT-2501) were cultured to 90% confluence in a T-75 flask (produced by SARSTEDT; product number: 89.3911.002) using BMCM-PL1 culture medium (produced by BIOFUTURE TECHNOLOGIES LTD.) containing 2% of human serum, were detached using trypsin-EDTA solution (produced by Nacalai Tesque, Inc.; product number: 32777-44), and were then cultured once again to confluence in a HYPERFlask (produced by Corning Incorporated) using 560 mL of BMCM-PL1 culture medium (produced by BIOFUTURE TECHNOLOGIES LTD.) containing 1% of human serum. The culture supernatant was collected. Thereafter, the culture supernatant was subjected to centrifugation to remove undissolved matter. Finally, the product was passed through a 0.45 µm filter (produced by Cytiva; product number: 6900-2504) to perform sterilization treatment and obtain a CM.

### (Example 2) Production of osteoblast progenitor cells by adhered culture

3 × 10⁵ cells/mL of ASCs (produced by Lonza; product number: PT-2501) were seeded and cultured to 20% to 30% confluence in a 10 cm dish (produced by SARSTEDT; product number: 83.3902) using 10 mL of a culture medium for mesenchymal stem cell proliferation (produced by Fukoku Co., Ltd.; product number: FKCM 301T).

Next, culturing was performed under each of the following culture conditions (1) to (4), the culture medium was subsequently switched to an osteoblast differentiation-inducing culture medium BMK-R008 (produced by BIOFUTURE TECHNOLOGIES LTD.), and then 2 days, 4 days, and 6 days of culturing were performed.
Culture conditions (1): Culture to 80% to 90% confluence
Culture conditions (2): Switch culture medium to IMDM (produced by Nacalai Tesque, Inc.; product number: 11506-05) in state of 80% to 90% confluence and then culture to confluence
Culture conditions (3): Culture to confluence
Culture conditions (4): Culture to hyperproliferation for 2 days from state at which confluence is reached

Next, ASCs that had been cultured in the 10 cm dish were detached and collected, were suspended in IMDM to prepare 1 × 10⁵ cells/mL/well, and were seeded in a 24-well plate (produced by SARSTEDT; product number: 83.3922).

The next day, the culture medium was switched to the following four types of culture media:
culture medium (A): IMDM,
culture medium (B): CM + IMDM (mixing ratio 1:1),
culture medium (C): CPPs composition + IMDM (mixing ratio 1:1),
culture medium (D): osteoblast differentiation-inducing culture medium (positive control),
and then 1 to 8 days of culturing was performed while replacing the culture medium. The CM and CPPs composition were those that were produced in Example 1 and Comparative Example 1 described above. Note that the mixing ratios given above are volume ratios.

After 1 to 8 days of culturing, an Alizarin Red S Staining Kit BMK-R009 (produced by BIOFUTURE TECHNOLOGIES LTD.) was used to fix each well of (1) to (4) and stain calcified nodules (calcium phosphate). Pigment bonded to calcium phosphate was extracted with 5% formic acid, and light absorbance was measured at OD 450 nm.

When there is red staining with Alizarin Red, the light absorbance at OD 450 nm of the pigment that has eluted into formic acid is 0.100 or more. The following describes an experiment providing a basis for this.

Cells that had been obtained by the culture conditions (4) (culture to hyperproliferation for 2 days from state at which confluence (100%) is reached), followed by a further 4 days of culturing with the culture medium switched to an osteoblast differentiation-inducing culture medium, were used to perform a further 3 to 8 days of culturing using the following culture media.
(a) CM + IMDM (mixing ratio 1:1)
(b) CPPs composition + IMDM (mixing ratio 1:1)
(c) Osteoblast differentiation-inducing culture medium (positive control)

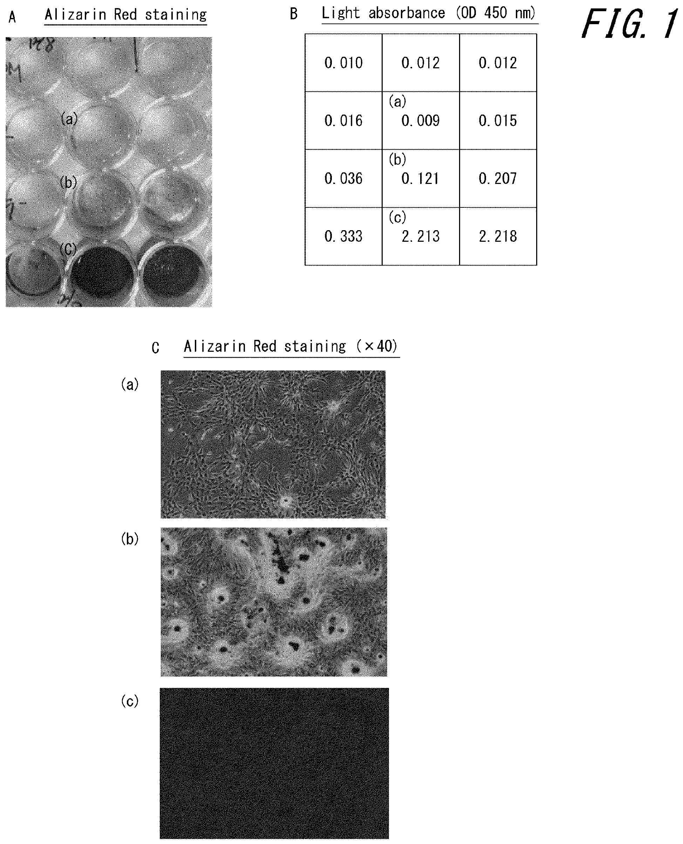

The results are presented in FIG. 1. In FIG. 1, A is a photograph of Alizarin Red staining (day 6 of culture), B presents measurement results of light absorbance at OD 450 nm (days 3, 6, and 8 of culture from left-hand column), and C is an enlarged micrograph (×40) of Alizarin Red staining (day 6 of culture).

With regard to (a), it can be seen through inspection of A(a) and C(a) in FIG. 1 that there is no staining. The light absorbance in this case is 0.009 as indicated by B(a) in FIG. 1.

With regard to (b), it can be seen through inspection of A(b) and C(b) in FIG. 1 that nodules (calcified spheres) are stained and that calcification has begun. The light absorbance in this case is 0.121 as indicated by B(b) in FIG. 1 and is approximately 10 times B(a).

(c) serves as a positive subject indicating a state in which calcification has fully progressed.

Based on these results, calcification is judged to have occurred when the light absorbance at OD 450 nm is 0.100 or more, and this was adopted as a criterion for judging that mesenchymal stem cells and osteoblast progenitor cells have been induced to differentiate into osteoblasts.

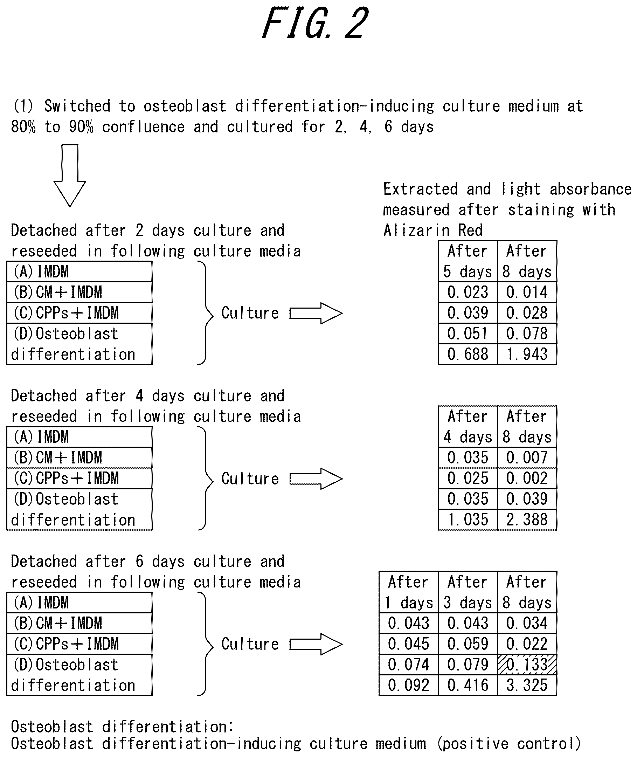

The tables in FIGS. 2 to 5 respectively present results for the culture conditions (1) to (4) described above.

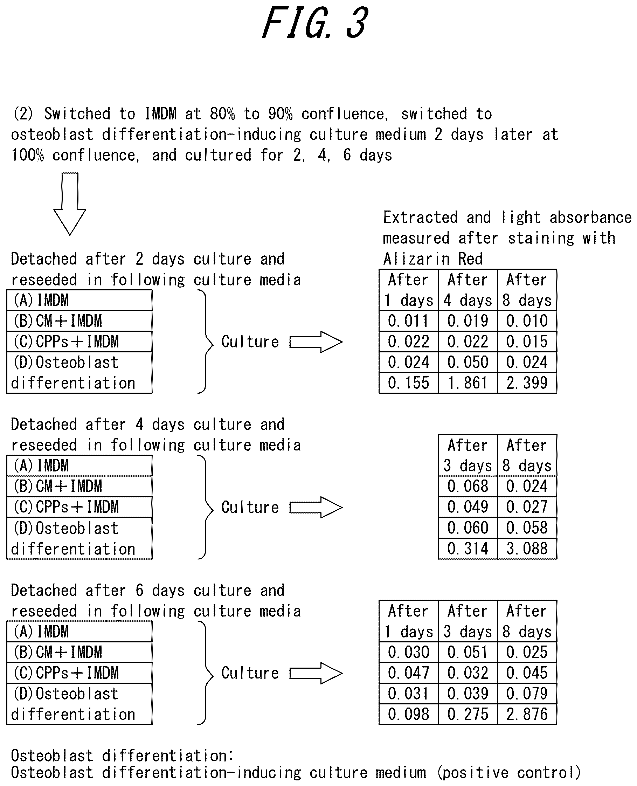

With the culture conditions (1) and (2), there was almost no calcification, and induction of differentiation into osteoblasts was not observed except with the culture medium (D) for the positive subject (FIG. 2 and FIG. 3).

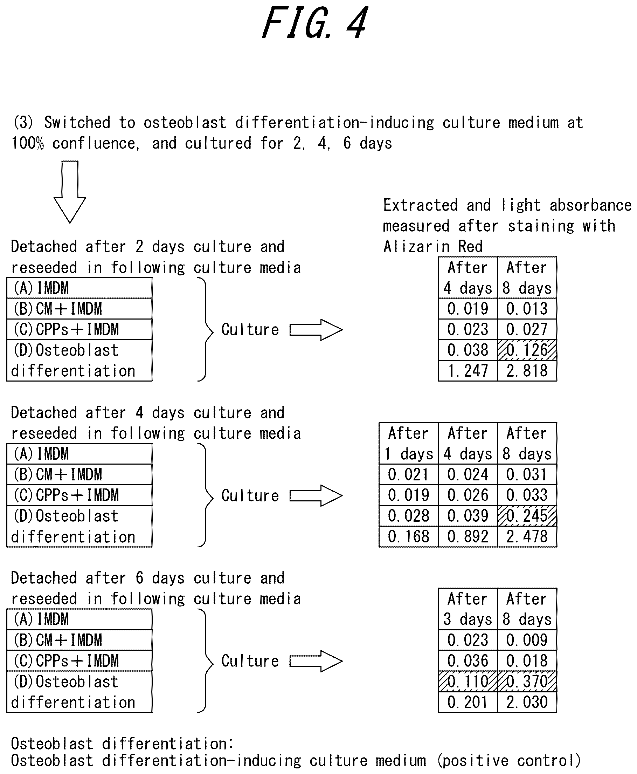

On the other hand, with the culture conditions (3), calcification occurred after 8 days and induction of differentiation into osteoblasts was confirmed in cases in which, at 2 days and 4 days after switching to osteoblast differentiation at confluence, cells were detached and seeded in a plate, and the culture medium was switched to the culture medium (C) (CPPs composition + IMDM), and calcification occurred after 3 days and after 8 days and induction of differentiation into osteoblasts was confirmed in a case in which, at 6 days after switching to the osteoblast differentiation-inducing culture medium at confluence, cells were detached and seeded in a plate, and the culture medium was switched to the culture medium (C) (FIG. 4).

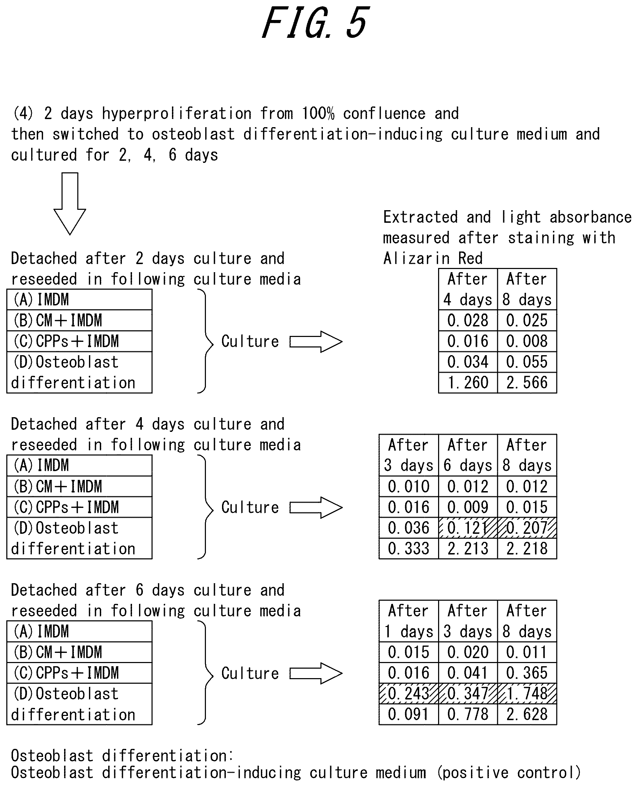

Moreover, with the culture conditions (4), calcification occurred after 6 to 8 days and after 1 to 8 days and induction of differentiation into osteoblasts was confirmed in cases in which, at 4 days and 6 days after switching to the osteoblast differentiation-inducing culture medium after 2 days of hyperproliferation from confluence, cells were detached and seeded in a plate, and the culture medium was switched to the culture medium (C) (FIG. 5).

As can be seen from FIGS. 2 to 5, calcification occurred after 3 days from seeding in a plate in a case in which culturing was performed using the culture medium (D) for the positive control, whereas calcification did not occur at all with any of the culture conditions (1) to (4) in a case in which culturing was performed using the culture medium (A) (IMDM) or the culture medium (B) (CM + IMDM). In contrast, in a case in which culturing was performed using the culture medium (C) (CPPs composition + IMDM), calcification was promoted according to the culture time, and induction of differentiation into osteoblasts was possible with the culture conditions (3) and (4).

Based on these results, it is thought that cells that had been induced to differentiate into osteoblast progenitor cells through the culture conditions (3) and (4) were further induced to differentiate into osteoblasts through culturing using the culture medium (C) (CPPs composition + IMDM).

### (Example 3) Production of osteoblast progenitor cells by suspension at rest

Next, a method of causing differentiation of MSCs into osteoblast progenitor cells at rest in a suspended state, without adhesion of the MSCs to a culture vessel, was investigated.

1 × 10⁶ cells/mL of ASCs (produced by Lonza; product number: PT-2501) were cultured in a T-175 flask (produced by SARSTEDT; product number: 83.3912.002) using 40 mL of a culture medium for mesenchymal stem cell proliferation FKCM 301T (produced by Fukoku Co., Ltd.). Next, 1.7 × 10⁶ cells were added into four 15 mL tubes (produced by SARSTEDT; product number: 62.554.5.2) together with the four types of culture media used in Example 2:
culture medium (A): IMDM,
culture medium (B): CM + IMDM (mixing ratio 1:1),
culture medium (C): CPPs composition + IMDM (mixing ratio 1:1),
culture medium (D): osteoblast differentiation-inducing culture medium (positive control),
in an amount of 4 mL and were blended. These cells were left at rest in a suspended state under refrigerated storage (4°C to 5°C) for 2 days, 4 days, and 6 days. Note that the mixing ratios given above are volume ratios.

After each refrigerated storage period, 10 µL was sampled from each tube, and the viable cell count was measured using a hemocytometer (produced by Erma Inc.; product number: 4025). Thereafter, the culture medium in each of the four tubes was removed by centrifugal separation, suspension was performed in IMDM (produced by Nacalai Tesque, Inc.; product number: 11506-05), and seeding was subsequently performed in a 48-well plate (produced by SARSTEDT; product number: 83.3923).

The viable cell (viability) ratio of ASCs counted by the viable cell count measurement described above was 84% to 89% on day 2, 88% to 93% on day 4, and 79% to 91% on day 6. The next day after seeding and adhesion in the 48-well plate, the culture medium was switched to the four types of culture media (A) to (D) described above. In this manner, culturing was performed under 16 sets of conditions, and staining with Alizarin Red was performed after 4 days and after 8 days in the same way as in Example 2.

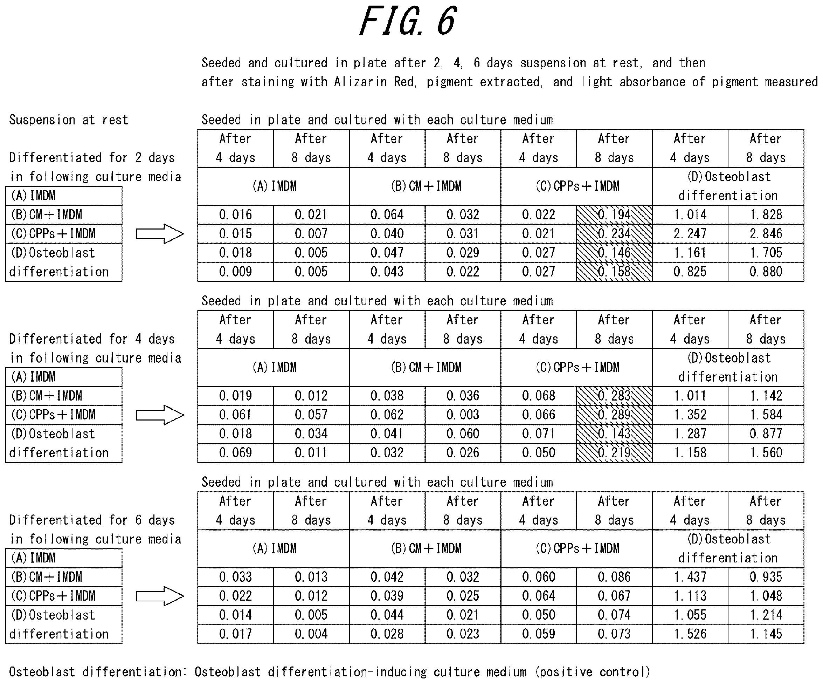

The results are presented in FIG. 6. In cases in which the refrigerated storage period in a suspended state was 2 days and 4 days, calcification was observed after 8 days of culture using the culture medium (C) (CPPs composition + IMDM). However, in a case in which the refrigerated storage period in a suspended state was 6 days, calcification was only observed with the culture medium (D) (osteoblast differentiation-inducing culture medium (positive control)). Calcification was not observed at all with the culture medium (A) (IMDM) and the culture medium (B) (CM + IMDM) under any of the culture conditions.

Based on the results described above, it is thought that after refrigerated storage in a suspended state for a short period, ASCs had been induced to differentiate into osteoblast progenitor cells, and subsequent culturing in a culture medium having the CPPs composition added thereto induced differentiation of osteoblast progenitor cells into osteoblasts. It was also found that even when the CPPs composition is not added to the culture medium during refrigerated storage in a suspended state, this does not affect subsequent induction of differentiation into osteoblasts.

### (Example 4) Verification of induction of differentiation into osteoblast progenitor cells by suspension at rest

2 × 10⁵ cells/mL of ASCs (produced by Lonza; product number: PT-2501) were seeded in a 24-well plate (produced by SARSTEDT; product number: 83.3922) containing 0.5 mL/well of the four culture media that were used in Example 2:
culture medium (A): IMDM,
culture medium (B): CM + IMDM (mixing ratio 1:1),
culture medium (C): CPPs composition + IMDM (mixing ratio 1:1),
culture medium (D): osteoblast differentiation-inducing culture medium (positive control),
and were cultured for 7 to 11 days without performing a step of refrigerated storage in a suspended state. Thereafter, Alizarin Red staining was performed in the same way as in Example 2. Note that the mixing ratios given above are volume ratios. The results are presented in Table 1. Calcification only occurred with the culture medium (D) for the positive control, and calcification was not observed with the culture media (A) to (C). This demonstrates that a step of refrigerated storage in a suspended state (step of placing at rest and performing 2 to 4 days of refrigerated storage in a suspended state in a culture vessel) prior to culture using a basal culture medium having a CPPs composition added thereto is an essential step for inducing differentiation into osteoblasts.

**[Table 1]**

| Culture medium | After 7 days | After 9 days | After 11 days |
|---|---|---|---|
| (A) IMDM | 0.040 | 0.061 | 0.029 |
| (B) CM + IMDM | 0.049 | 0.086 | 0.070 |
| (C) CPPs composition + IMDM | 0.050 | 0.057 | 0.044 |
| (D) Osteoblast differentiation-inducing culture medium (positive control) | 1.328 | 1.650 | 2.108 |

### (Example 5) CPPs composition analysis 1

In order to confirm the characteristics of the CPPs composition, the type I collagen concentration, hyaluronic acid concentration, and collagen and Ca were measured, and a comparative investigation was made with the reference culture supernatant (CM).

The type I collagen concentration in the CPPs, as measured by an ELISA (prepared ELISA corresponding to a human collagen type 1 ELISA (produced by ACEL, Inc.; product number: EC1-E105)) and SDS-PAGE (performed according to the revised 4th edition of "Protein Experiment Notebook (Vol. 2)", Yodosha Co., Ltd.), was 60 µg/mL to 100 µg/mL and had an average of 80 µg/mL.

The hyaluronic acid concentration in the CPPs, as measured using a hyaluronic acid quantification kit (produced by Cosmo Bio Co., Ltd.; product number: HA-96KIT), was 140 µg/mL.

TGF-β1 in the CPPs, as measured using a human TGF-β1 ELISA kit (produced by Bioss Inc.; product number: BSKH1021) was 384 pg/mL to 810 pg/mL.

The same measurements were performed for the CM, and these measurements were compared. The results are presented in Table 2.

**[Table 2]**

| | Type I collagen (µg/mL) | Hyaluronic acid (µg/mL) | TGF-β1 (pg/mL) |
|---|---|---|---|
| CM | 1.9 | 2.2 | 260 |
| CPPs composition | 80 | 110 | 384 to 810 |

Upon comparison of the CPPs composition with the CM, which is a typical culture supernatant, it can be seen that the type I collagen concentration is approximately 42 times higher, the hyaluronic acid concentration is approximately 50 times higher , and TGF-β1 is at most 3.1 times higher in the CPPs composition and that the CPPs composition is rich in these components.

Moreover, based on the results presented up to here, it is clear that the CPPs composition has an effect of calcification promotion activity in the same manner as an osteoblast differentiation-inducing culture medium and is highly likely to contain type I collagen and Ca that are essential for calcification. In Example 5, since a high type I collagen concentration had been confirmed, the Ca concentration of the CPPs composition was also measured by metallo assay calcium measurement (OCPC) (produced by Metallogenics Co., Ltd.; product number: CA30M) and was determined to be 40 µg/mL. However, the Ca concentration of the CPPs composition should, in theory, be almost 0 µg/mL because Ca does not sediment in the organic solvent and is not contained in hydrochloric acid and caustic soda used for virus inactivation treatment of sedimented protein in production of the CPPs composition.

Therefore, Ca was predicted to be bonded to type I collagen in the CPPs composition.

### (Example 6) CPPs composition analysis 2

The prediction described above was confirmed through the following measurement.

First, in order to eliminate the influence of non-specific Ca adsorption, 10 mL of the CPPs composition was concentrated to approximately 1.7 mL using a centrifugal concentrator (produced by Corning Incorporated, Spin-X UF20). The Ca concentration of the concentrated sample, as measured by the metallo assay calcium measurement described above, was 174 µg.

Thereafter, 10.3 mL of physiological saline was added to the concentrated sample, and the sample was concentrated to 1.5 mL using the aforementioned centrifugal concentrator. The Ca (calcium) concentrations of the filtrate and the concentrate were measured.
Ca concentration in filtrate (10.5 mL): 4.1 µg/mL
Ca concentration in concentrate (1.5 mL): 38.8 µg/mL

On the other hand, the type I collagen concentrations of the filtrate and the concentrate were measured by the previously described ELISA.
Type I collagen concentration in filtrate: 0.1 µg/mL or less
Type I collagen concentration in concentrate: 360 µg/mL

It can be seen from the results presented above that there is correlation between the type I collagen concentration and the Ca concentration in the concentrate and that type I collagen and Ca behave in the same manner. In other words, the result that the majority of Ca remains in the concentrate without being filtered indicates that Ca in the culture medium is bonded to type I collagen in the CPPs composition.

### (Example 7) CPPs composition analysis 3

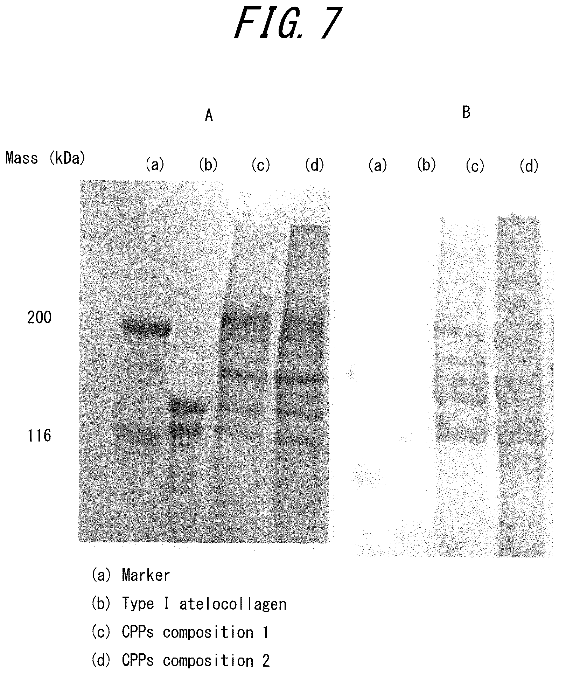

After the CPPs composition had undergone electrophoresis by 10% gel SDS-PAGE with type I atelocollagen (produced by Daiichi Fine Chemical Co., Ltd.; product number: Y-1), the CPPs composition was transferred to a nitrocellulose membrane and was subjected to western blotting (performed according to the revised 4th edition of "Protein Experiment Notebook (Vol. 2)", Yodosha Co., Ltd.). The results are presented in FIG. 7. In FIG. 7, A is results of CBB staining (produced by Nacalai Tesque, Inc.; product number: 04543-51) and B is an electrophoresis pattern obtained through reaction with Anti-Procollagen 1C-Terminal Propeptide (Anti-PICP) Antibody (produced by Cloud-Clone Corp.; product number: PAA570Hu08).

Type I atelocollagen appeared as two bands at approximately 120 kDa (mass) in CBB staining as illustrated in A(b) in FIG. 7, but did not react with anti-PICP antibody, which marks propeptide C-terminals, and thus a band was not detected as illustrated in B(b) in FIG. 7.

On the other hand, with CPPs compositions indicated by (c) and (d) in FIG. 7, at least 5 bands were detected at approximately 120 kDa to 200 kDa. These results indicate that procollagen (collagen precursor) is included among type I collagen in the CPPs composition.

The procollagen that is contained in the CPPs composition forms a composite with calcium and can thereby serve as a cell scaffolding and facilitate the supply of calcium to cells.

Through a CPPs composition having features such as set forth above, it is possible to induce differentiation into osteoblasts at a desired timing through localized administration of the CPPs composition at the same time as osteoblast progenitor cells or through subsequent administration of the CPPs composition as a booster. Moreover, it is also possible to induce differentiation of mesenchymal stem cells and osteoblast progenitor cells into osteoblasts using components obtained from mesenchymal stem cells and components derived from autologous cells, without using an osteoblast differentiation-inducing culture medium.

## Claims

1. A culture medium for stem cell culture comprising:
60 vol% to 90 vol% of a basal culture medium; and
10 vol% to 40 vol% of physiological saline, and further having, added thereto:
1 ng/mL to 100 ng/mL of EGF;
0.2 ng/mL to 20.0 ng/mL of FGF-2;
0.2 ng/mL to 20.0 ng/mL of PDGF; and
0.5 mM to 8.0 mM of magnesium ascorbyl phosphate.

2. A cell producing proteins (CPPs) composition for inducing differentiation of mesenchymal stem cells or osteoblast progenitor cells into osteoblasts, wherein the CPPs composition is obtained by:
culturing mesenchymal stem cells in the culture medium for stem cell culture according to claim 1 for 1 to 10 days;
subsequently causing sedimentation of protein components in an organic solvent; and
separating a sedimented portion and dissolving the sedimented portion in physiological saline or a basal culture medium.

3. The CPPs composition according to claim 2, comprising:
type I procollagen; and
hyaluronic acid, wherein
calcium is bonded to the type I procollagen.

4. A method of inducing differentiation into osteoblasts comprising:
causing differentiation of mesenchymal stem cells into osteoblast progenitor cells by
1) culturing the mesenchymal stem cells for 2 to 6 days in an osteoblast differentiation-inducing culture medium with the mesenchymal stem cells in a state adhered to a culture vessel and causing proliferation until confluence or beyond, or
2) placing the mesenchymal stem cells at rest and performing 2 to 4 days of refrigerated storage with the mesenchymal stem cells in a suspended state in a culture vessel; and
subsequently collecting the osteoblast progenitor cells, seeding the osteoblast progenitor cells in a culture vessel, and culturing the osteoblast progenitor cells in a basal culture medium having the CPPs composition according to claim 2 added thereto to cause differentiation of the osteoblast progenitor cells into osteoblasts.

5. The method of inducing differentiation into osteoblasts according to claim 4, wherein the mesenchymal stem cells are derived from bone marrow, adipose tissue, peripheral blood, umbilical cord, umbilical cord blood, or dental pulp.

6. A method of inducing differentiation into osteoblast progenitor cells comprising causing differentiation of mesenchymal stem cells into osteoblast progenitor cells by:
1) culturing the mesenchymal stem cells for 2 to 6 days in an osteoblast differentiation-inducing culture medium with the mesenchymal stem cells in a state adhered to a culture vessel and causing proliferation until confluence or beyond; or
2) placing the mesenchymal stem cells at rest and performing 2 to 4 days of refrigerated storage with the mesenchymal stem cells in a suspended state in a culture vessel.

7. The method of inducing differentiation into osteoblast progenitor cells according to claim 6, wherein the mesenchymal stem cells are derived from bone marrow, adipose tissue, peripheral blood, umbilical cord, umbilical cord blood, or dental pulp.

8. A method of inducing differentiation into osteoblasts comprising:
seeding osteoblast progenitor cells that have been obtained by the method of inducing differentiation according to claim 6 and simultaneously adding the CPPs composition according to claim 2 to cause differentiation of the osteoblast progenitor cells into osteoblasts; or
seeding osteoblast progenitor cells that have been obtained by the method of inducing differentiation according to claim 6 and subsequently adding the CPPs composition according to claim 2 to initiate differentiation of the osteoblast progenitor cells into osteoblasts.
